# EUROPEAN PATENT SPECIFICATION

(11) **EP 2 346 481 B1**
(45) Date of publication and mention of the grant of the patent: **31.08.2016**
(21) Application number: 09740069.1
(22) Date of filing: 20.10.2009
(51) Int. Cl.: A61K 8/06, A61Q 5/04, A61K 8/895, A61K 8/41, A61K 8/42

(54) **COMPOSITION FOR THE PERMANENT SHAPING OF HUMAN HAIR**
ZUSAMMENSETZUNG ZUR DAUERHAFTEN VERFORMUNG DES MENSCHLICHEN HAARES
COMPOSITION DE MISE EN FORME PERMANENTE DE CHEVEUX HUMAINS

(30) Priority: 20.10.2008 EP 08018288
(43) Date of publication of application: 27.07.2011
(73) Proprietor: Kao Germany GmbH, 64297 Darmstadt (DE)
(72) Inventor: WOOD, Jonathan, 69469 Weinheim (DE); HULLMANN, Alexandra, 64331 Weinheim (DE); GRIT, Mustafa, 64579 Gernsheim (DE)
(74) Representative: Grit, Mustafa
(86) International application number: PCT/EP2009/007502
(87) International publication number: WO 2010/046080

(56) References cited:
- EP-A- 0 874 017
- EP-A- 1 676 567
- EP-A- 1 797 861
- EP-A- 1 880 709
- EP-A- 1 894 556
- GB-A- 2 197 352
- US-A1- 2006 083 704
- US-B1- 6 451 298
- "DOW CORNING HMW 2220 NON-IONIC EMULSION", INTERNET CITATION, [Online] XP002232355, Retrieved from the Internet: URL:http://wwwgalindberg.se/produktdata.ht m> [retrieved on 2003-02-24]

## Description

The present invention concerns a composition for the permanent shaping of human hair used both for the permanent waving of human hair with an excellent waving effect as well as for the straightening of either naturally or chemically curled hair.

It is generally known that permanent waving is carried out in two steps, the reductive splitting of the cysteine disulfide bonds in the hair by a reducing agent and the subsequent neutralization by application of an oxidizing agent, whereby the cysteine disulfide bonds are restored.

The reducing agent still most frequently used today is thioglycolic acid, also in form of the salts thereof, in particular its ammonium salt, although numerous other thio compounds have been proposed for this purpose, which, however, mostly did not succeed.

The compositions containing thioglycolates are customarily applied at a pH-value between 7 and 10, in particular 8.5 and 9.5.

Such compositions vary in their waving and/or straightening performance and, therefore, there is still need for further improvement, especially in permanent shaping of damaged to strongly damaged hair and in particular for permanent shaping hair streaks including parts with various damage level in length.

Cationic or cationizable surfactants have commonly been used in permanent shaping compositions for hair. The ones used are alkyl amine or alkyl quaternary amine types and not the ones described in the present invention.

The present invention starts from the task of providing a composition for the permanent shaping of human hair with excellent waving and straightening performance. Hair waved or straightened with composition disclosed herein looks and feels natural upon touching by hand. For waved hair it is especially important that the hair has excellent elasticity and bounce.

It has surprisingly been found our by the inventors of the present invention that a permanent shaping composition for hair based on at least one reducing agent further comprising an aqueous emulsion of a high viscosity silicone copolymer and at least one alkyl ether or ester or amine alkyl amine or quaternary ammonium compound shows excellent waving effect and further hair feels natural upon touching and excellent shine, elasticity and bounce.

Accordingly, the first object of the present invention is an aqueous composition for permanent shaping and/or straightening hair based on at least one reducing agent and further comprising an aqueous emulsion of divinyldimethicone/dimethicone copolymer with an internal phase viscosity of more than 1 x 10⁸ mm²/s measured at 0.01 Hz and at about 25°C and at least one compound according to general structure

R₁-A-R₂-B

wherein R₁ is a saturated or unsaturated, straight or branched alkyl group with 8 to 24 C atoms, R₂ is a straight or branched alkyl group with 1 to 4 C atoms, A is a group selected from O, 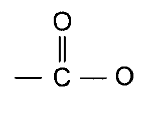 and 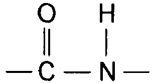 and B is selected from 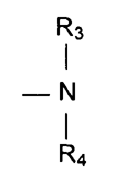 wherein R₃ and R₄ are the same or different is H or an alkyl with 1 to 4 C atoms, hydroxyl alkyl with 1 to 4 C atoms and di hydroxyl alkyl with 2 to 4 C atoms, 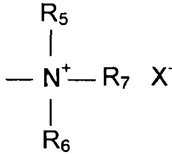 R₅, and R₆ are the same or different, an alkyl with 1 to 4 C atoms, hydroxyl alkyl with 1 to 4 C atoms and di hydroxyl alkyl with 2 to 4 C atoms, R₇ is an alkyl with 1 to 4 C atoms, hydroxyl alkyl with 1 to 4 C atoms or di hydroxyl alkyl with 2 to 4 C atoms and

-R₂-A-R₁

wherein R₁, A and R₂ have the above meaning and X is chloride, bromide, methosulfate.

Aqueous composition of the present invention comprise aqueous emulsion of divinyldimethicone/dimethicone copolymer with a viscosity of more than 1 x 10⁸ mm²/s, preferably 1.1 x 10⁸ mm²/s, and more preferably 1.2 x 10⁸ mm²/s. Divinyldimethicone/dimethicone copolymer is comprised in compositions of the present invention at a concentration of 0.01 to 5% by weight, preferably 0.02 to 3%, more preferably 0.05 to 2.5% by weight and most preferably 0.1 to 1.5% by weight calculated to total composition as Divinyldimethicone/dimethicone copolymer.

In a preferred embodiment of the present invention, aqueous divinyldimethicone/dimethicone copolymer emulsion comprises non-ionic surfactants and dispersed droplet has an average droplet size of smaller than 0.6 µm. Suitable divinyldimethicone/dimethicone copolymer emulsion with an internal phase viscosity at 0.01 Hz more than 1.2 x 108 is available from Dow Corning under the trade name HMW 2220. The non-ionic emulsion comprises C12-13 Pareth-23 and C12-13 Pareth-3 as non-ionic emulsifiers.

All concentration mentioned within the description refers to the concentration of the respective compound in the composition prior to mixing with any other composition, if necessary, unless otherwise mentioned.

Compositions of the present invention comprise at least one alkyl ether alkyl amine or alkyl ether alkyl quaternary amine or alkyl ester/amide alkyl amine or alkyl ester/amide alkyl quaternary amine according to the above general structures. In the preferred embodiment of the present invention, R₁ is saturated or unsaturated, straight or branched alkyl group with 10 to 24C atoms, more preferably 12 to 22 C atoms and R₂ is straight or branched alkyl group with 1 to 4 C atoms, A, B, R₃ to R₇ are same as above. Non-limiting suitable examples are stearyloxypropyl amine, palmityloxypropyl amine, stearyloxypropyldimethyl amine, stearyloxypropyldiethyl amine, stearyloxyethylyldimethyl amine, stearyloxyethyl amine, myristyloxypropyl amine, myristyloxypropyldimethyl amine, palmitamidopropyl amine, palmitamidopropyl methylamine, palmitamidopropyl diethylamine, palmitamidopropyl dibutylamine, palmitamidopropyl buylamine, palmitamidopropyl dipropylamine, palmitamidopropyl propylamine, palmitamidopropyl dihydroxyethylamine, palmitamidopropyl hydroxyethylamine, palmitamidopropyl dihydroxypropylamine, palmitamidopropyl hydroxypropylamine, lauramidopropyl amine, lauramidopropyl methylamine, lauramidopropyl diethylamine, lauramidopropyl dibutylamine, lauramidopropyl buylamine, lauramidopropyl dipropylamine, lauramidopropyl propylamine, lauramidopropyl dihydroxyethylamine, lauramidopropyl hydroxyethylamine, lauramidopropyl dihydroxypropylamine, lauramidopropyl hydroxypropylamine, stearamidopropyl amine, stearamidopropyl methylamine, stearamidopropyl diethylamine, stearamidopropyl dibutylamine, stearamidopropyl butylamine, stearamidopropyl dipropylamine, behenamidopropyl propylamine, behenamidopropyl dihydroxyethylamine, behenamidopropyl hydroxyethylamine, behenamidopropyl dihydroxypropylamine, behenamidopropyl hydroxypropylamine, behenamidopropyl amine, behenamidopropyl methylamine, behenamidopropyl diethylamine, behenamidopropyl dibutylamine, behenamidopropyl butylamine, behenamidopropyl dipropylamine, behenamidopropyl propylamine, behenamidopropyl dihydroxyethylamine, behenamidopropyl hydroxyethylamine, behenamidopropyl dihydroxypropylamine, behenamidopropyl hydroxypropylamine, dipalmitamidopropyl methylamine, dipalmitamidopropyl ethylamine, dipalmitamidopropyl butylamine, dipalmitamidopropyl propylamine, dipalmitamidopropyl hydroxyethylamine, dipalmitamidopropyl hydroxypropylamine, dilauramidopropyl amine, dilauramidopropyl methylamine, dilauramidopropyl buylamine, dilauramidopropyl hydroxyethylamine, dilauramidopropyl hydroxypropylamine, distearamidopropyl amine, distearamidopropyl methylamine, dibehenamidopropyl propylamine, dibehenamidopropyl hydroxyethylamine, palmitoamidopropyl trimethyl ammonium chloride, stearamidopropyl trimethylammonium chloride, behenamidopropyl tri hydroxyethalmonium chloride, distearylamidopropyl dimethyl ammonium chloride, dicetylamidodihydroxyethyl ammonium chloride, palmitoylpropyl amine, palmitoylpropyl methylamine, palmitoylpropyl diethylamine, palmitoylpropyl dibutylamine, palmitoylpropyl buylamine, palmitoylpropyl dipropylamine, palmitoylpropyl propylamine, palmitoylpropyl dihydroxyethylamine, palmitoylpropyl hydroxyethylamine, palmitoylpropyl dihydroxypropylamine, palmitoylpropyl hydroxypropylamine, myristoylpropyl amine, myristoylpropyl methylamine, myristoylpropyl diethylamine, myristoylpropyl dibutylamine, myristoylpropyl buylamine, myristoylpropyl dipropylamine, myristoylpropyl propylamine, myristoylpropyl dihydroxyethylamine, myristoylpropyl hydroxyethylamine, myristoylpropyl dihydroxypropylamine, myristoylpropyl hydroxypropylamine, stearoylpropyl amine, stearoylpropyl methylamine, stearoylpropyl diethylamine, stearoylpropyl dibutylamine, stearoylpropyl butylamine, stearoylpropyl dipropylamine, behenoylpropyl propylamine, behenoylpropyl dihydroxyethylamine, behenoylpropyl hydroxyethylamine, behenoylpropyl dihydroxypropylamine, behenoylpropyl hydroxypropylamine, behenoylpropyl amine, behenoylpropyl methylamine, behenoylpropyl diethylamine, behenoylpropyl dibutylamine, behenoylpropyl butylamine, behenoylpropyl dipropylamine, behenoylpropyl propylamine, behenoylpropyl dihydroxyethylamine, behenoylpropyl hydroxyethylamine, behenoylpropyl dihydroxypropylamine, behenoylpropyl hydroxypropylamine, dipalmitoylpropyl methylamine, dipalmitoylpropyl ethylamine, dipalmitylpropyl butylamine, dipalmitylpropyl propylamine, dipalmitylpropyl hydroxyethylamine, dipalmitylpropyl hydroxypropylamine, dilauroylpropyl amine, dilauroylpropyl methylamine, dilauroylpropyl buylamine, dilauroylpropyl hydroxyethylamine, dilauroylpropyl hydroxypropylamine, distearylpropyl amine, distearylpropyl methylamine, dibehenylpropyl propylamine, dibehenylpropyl hydroxyethylamine, palmitylpropyl trimethyl ammonium chloride, stearylpropyl trimethylammonium chloride, behenylpropyl tri hydroxyethalmonium chloride, distearylpropyl dimethyl ammonium chloride, dicetyldihydroxyethyl ammonium chloride, dioleoylethylhydroxyethylmonium methosulfate, and dicocoylethylhydroxyethylmonium methosulfate.

Concentration of at least one alkyl ester/amide alkyl amine or alkyl ester/amide alkyl quaternary amine according to the above general structure is in the range of 0.01 to 5%, preferably 0.02 to 4%, more preferably 0.05 to 3% and most preferably 0.1 to 2.5% and in particular 0.2 to 2% by weight calculated to total composition.

The permanent shaping compositions according to the invention comprise at least one reducing compound at a concentration of at least 0.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1.2-propyleneglycol monothioglycolate (see also WO-A 93/1791), 1-3-propanediol monothioglycolate or the isomer mixture resulting thereof, 1.3-butanediol and 1.4-butanediol monothioglycolate and the isomer mixtures thereof, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 0.5 to 15 %, preferably 2.0 to 12.5% by weight, calculated to total composition as free thioglycolic acid as reference substance.

The permanent shaping compositions containing reducing agents can, if necessary, comprise alkalizing agents. Their quantity is dependent on the reducing agent and the desired pH-value of the composition. Reducing agent compositions preferably contain 0.1% to 5%, in particular 0.5% to 2.5% by weight thereof, calculated to the total composition. Alkalizing agents preferred within the scope of the invention are ammonium carbamate, ammonia and/or ammonium(bi)carbonate, monoethanolamine and triethanolamine. It is desirable to adjust the pH-value between about 6.5 and 10.5, preferably about 7 to 9.5.

The permanent shaping compositions according to the invention are suited for use both for the permanent waving, i.e. curling of human hair and for the straightening, i.e. smoothing thereof.

The viscosity best suited for the permanent shaping compositions according to the invention proved to be in the range of 1 to 10,000 mPa.s, preferably about 1 to about 5,000 mPa.s, measured at 20°C in a Brookfield viscosimeter (no. 5 spindle), whereas the viscosity suited for the straightening compositions is preferably higher in a range up to 50,000 mPa.s, preferably up to 30,000 mPa.s measured at 20°C in a Brookfield viscosimeter (no. 5 spindle).

The viscosity is adjusted by addition of the appropriate amounts of thickening agents known per se, such as cellulose derivatives. Thickening may as well be realized by formulating a composition in form of an emulsion with the use of C₁₀-C₂₂-fatty alcohols, in mixture with long mono alkyl chain quaternary ammonium surfactants.

The permanent shaping compositions according to the present invention preferably comprise surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, permanent shaping compositions comprise additionally at least one cationic surfactant according to general formula 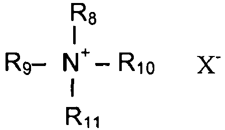 where R₈ is a saturated or unsaturated, branched or non-branched alkyl chain with 8-24 C atoms R₉ is a hydrogen, saturated or unsaturated, branched or non-branched alkyl chain with 1-24 C atoms and R₁₀ and R₁₁ are independent from each other H or lower alkyl chain with 1 to 4 carbon atoms which may be substituted with one or two hydroxyl group, and X is chloride, bromide or methosulfate.

Concantration of cationic surfactant is in the range from 0.05 % to 5 %, preferably 0.1 % to 2.5 % by weight, calculated to total composition.

Non-limiting suitable long-chain quaternary ammonium compounds are in particular cetyl trimethyl ammonium chloride, dimethyl dicetyl ammonium chloride, trimethyl cetyl ammonium bromide, stearyl trimethyl ammonium chloride, dimethyl stearyl hydroxyethyl ammonium chloride, lauryl trimethyl ammonium chloride etc.

Further permanent shaping compositions of the present invention may comprise additional cationic polymer. Basically suitable are all cationic polymers listed under the generic name "Polyquaternium" in the CTFA International Cosmetic Ingredient Dictionary. Examples are Polyquaternium 2, Polyquaternium 4, Polyquaternium 5, Polyquaternium 6, Polyquaternium 7, Polyquaternium 10, Polyquaternium 11, Polyquaternium 16, Polyquaternium 22, Polyquaternium 28, Polyquaternium 39 and Polyquaternium 87.

The cationic polymers also include the quaternized products of graft polymers from organopolysiloxanes and polyethyl oxazolines described in EP-A 524 612 and EP-A 640 643. Such polymer is known with its CTFA name Polysilicone-9.

Concantration of one or more additional cationic polymers is in the range from 0.05 % to 2.5 %, preferably 0.1 % to 1.5 % by weight, calculated to total composition.

Permanent shaping compositions of present invention can comprise additionally at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

Permanent shaping composition of the present invention can comprise further ceramide type of compound such as cetyl-PG-hydroxyethylpalmitamide.

Further optional ingredient are sterols,especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C₁₀ to C₂₂ may be incorporated into the compositions of the present invention at a concentration of preferably 0.01 to 2.5% by weight calculated to total composition.

Another preferred compound in the permanent shaping composition of the present invention is silicone compounds and especially aminated silicones such as amodimethicone available from for example Dow Corning under the brand names Dow Corning 949 Emulsion and Dow Corning 2-8194. Concentration of silicones, especially amodimethicone, is in the range of 0.05 to 2.5%, preferably 0.1 to 1% by weight calculated to total composition.

Additionally, one or more natural oil component may be incorporated into the compositions of the present invention. Suitable are such as olive oil, almond oil, avocado oil, wheatgerm oil, ricinus oil or their mixture. Concentration of these natural oil ingredients should be 0.01 to 2.5%, preferably 0.01 to 1%, more preferably 0.05 to 0.5% by weight, calculated to total composition.

Further additional compounds may be present in the permanent shaping compositions of the present invention is ubichinone of the formula 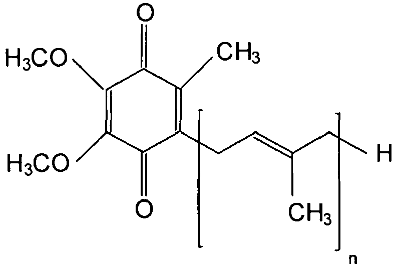 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in permanent shaping compositions of the present invention is from 0.0001 to 1%, preferably from 0.0002 to 0.75%, more preferably from 0.0002 to 0.5% and most preferably from 0.0005 to 0.5% by weight, calculated to total composition.

In another preferred embodiment of the present invention, composition comprises one or more fatty alcohol with a chain length of 12 to 24 C atoms. Concentration of one or more fatty alcohol is in the range of 1 to 15%, preferably 1 to 10%, more preferably 2 to 7.5 and most preferably 2 to 5% by weight calculated to total composition.

Suitable non limiting examples are lauryl alchohol, myristyl alcohol, cetyl alcohol, stearyl alcohol cetearyl alcohol, behenyl alcohol and their mixtures. Preferred are cetyl alcohol, stearyl alcohol cetearyl alcohol, behenyl alcohol and their mixtures. Particularly preferred are cetyl alcohol, stearyl alcohol and their 1 to 1, by weight, mixture cetearyl alcohol.

The compositions used according to the invention can naturally comprise all the substances customarily found in permanent shaping compositions, a list of which will not be given here, and are preferably present as solutions, gels with a higher or lower viscosity, emulsions or creams. They can be single-phase products or compositions packed into separate packaging which are united upon application, as they are disclosed, for example, in DE-C 43 04 828.

In order to avoid repetition, reference is here made to the state of the art as it is described, for example, in "Ullmann's Encyclopedia of Industrial Chemistry", Vol. A12 (1986), pages 588 to 591, and in particular to the monography of K. Schrader, "Grundlagen und Rezepturen der Kosmetika", 2nd. Ed. (1989, Hüthig Buchverlag) pages 823 to 840, as well as the article by D. Hollenberg et. al. in "Seifen-Öle-Fette-Wachse", 117 (1991), pages 81 to 87.

Composition of the present invention is used in a process for permanent waving wherein hair is washed or shampooed first and wound on the curlers, subsequently a reducing agent composition comprising at least one reducing agent, an aqueous emulsion of a high viscosity silicone copolymer disclosed above and at least one alkyl ether or ester or amide alkyl amine or quaternary ammonium compound according to general structures given above is applied onto hair and after 1 to 30 min, preferably 1 to 20 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers removed from hair. In cases where additional conditioning composition is required this may as well be applied after finishing the process as described above.

In another process as described above the curlers are removed after rinsing off the reducing agent and before applying the oxidizing agent.

Further in another process, after rinsing off the reducing agent from hair, an intermediate treatment composition is applied onto hair, and without rinsing off but after removing the excess amount of intermediate treatment with a towel, oxidizing composition is applied and at the end of the processing they are rinsed off from hair and curlers are removed from hair.

It has further been found out that the use of an aqueous emulsion of a high viscosity silicone copolymer and at least one amine/quaternary ammonium compound according to above given general structure in the intermediate treatment composition improves the permanent shaping of hair as well in terms of curl appearance and natural look and feel of hair. Therefore, in another preferred form of the present invention, permanent shaping of hair is carried our with a process wherein hair is washed or shampooed first and subsequently a reducing agent comprising composition is applied onto hair and after 1 to 45 min, preferably 1 to 30 min of processing time, depending on the hair strength, rinsed off from hair with tap water and an intermediate treatment composition comprising an aqueous emulsion of a high viscosity silicone copolymer and at least one alkyl ether or ester or amine alkyl amine or quaternary ammonium compound according to above given general structure is applied and without rinsing off an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off. In cases where additional conditioning composition is required this may as well be applied after finishing the above process. In case of permanent waving hair in the above process curlers are put onto hair before application of reducing composition and taken off from hair before application of oxidizing composition or after application and processing of the oxidizing composition.

The intermediate treatment composition has a pH value between 2.5 to 6, preferably 3 to 5.5 and most preferably 3 to 5.

A straightening process may also be carried out in a different process wherein hair is washed and/or shampooed and dried and reducing composition comprising at least one reducing compound, an aqueous emulsion of a high viscosity silicone copolymer and at least one alkyl ether or ester or amine alkyl amine or quaternary ammonium compound according to above given general structure is applied onto dry hair and processed for 5 to 60 min, preferably 5 to 45 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

The following examples are to illustrate the invention, but not to limit it.

### Example 1:

| | |
|---|---|
| Ammonium thioglycolate (60%) | 21.3 (% by wt. |
| Ammonium hydrogen carbonate | 5.0 |
| 1,3- butylene gylcol | 3.0 |
| Dow Corning HMW 2220 | 0.5 |
| Stearamidopropyl dimethyl amine | 0.5 |
| PEG-40-Hydrogenated castor oil | 0.7 |
| Perfume | 0.4 |
| Ammonia, 25% | ad pH 8.3 |
| Water | ad 100.0 |

With this composition the hair was permanently waved for about 15 minutes, rinsed and neutralized for about 8 minutes with a customary 2.5% H₂0₂ composition. Homogeneous wave appearance was obtained. Exclusion of Dow Corning HMW 2220 and stearamidopropyl dimethyl amine resulted in less homogeneous perm appearance.

### Example 2:

| | |
|---|---|
| Ammonium thioglycolate (60%) | 20 (% by wt. |
| Ammonium hydrogen carbonate | 4.0 |
| 1,3- butylene gylcol | 3.0 |
| Dow Corning HMW 2220 | 0.25 |
| Stearamidopropyltrimonium methosulfate | 0.3 |
| PEG-40-Hydrogenated castor oil | 0.7 |
| Perfume | 0.4 |
| Ammonia, 25% | ad pH 8.3 |
| Water | ad 100.0 |

With this composition the hair was permanently waved for about 18 minutes, rinsed and neutralized for about 10 minutes with a customary 2.5% H₂0₂ composition. Homogeneous wave appearance was obtained. Exclusion of Dow Corning HMW 2220 stearamidopropyltrimonium methosulfate resulted in less homogeneous perm appearance.

### Example 3:

**Alkaline Permanent Wave for Damaged Hair**

| | |
|---|---|
| Ammonium thioglycolate (60%) | 15.0 (% by wt.) |
| Ammonium hydrogen carbonate | 2.5 |
| Ceteth-20 | 0.7 |
| Cetrimonium chloride | 0.1 |
| Stearoylpropyltrimonium chloride | 0.25 |
| Dow Corning HMW 2220 | 0.8 |
| 1,3- butylene gylcol | 0.5 |
| Perfume | 0.4 |
| Ammonia, 25% | ad pH 8.0 |
| Water | ad 100.0 |

The permanent wave achieved with this composition was similar to the one obtained with the composition according to Example 1.

Exclusion of the Dow Corning HMW 2220 and stearoylpropyltrimonium chloride led to waves with substantially weaker contours.

### Example 4:

| | |
|---|---|
| Ammonium thioglycolate (60%) | 0.9 (% by wt.) |
| Cystein hydrochloride | 5.7 |
| Ammonium hydrogen carbonate | 1.5 |
| Acetylcystein | 0.7 |
| Cetrimonium chloride | 0.1 |
| 1,3- butylene gylcol | 0.5 |
| Dow Corning HMW 2220 | 1.2 |
| Palmitamidopropyl trimonium chloride | 0.5 |
| Amodimethicone | 0.2 |
| Coenzyme Q10 | 0.05 |
| Oleic acid | 0.05 |
| Perfume | 0.4 |
| Ammonia, 25% | ad pH 9.8 |
| Water | ad 100.0 |

The permanent wave achieved with this composition was similar to the one obtained with the composition according to Example 1.

Exclusion of the Dow Corning HMW 2220 and palmitamidopropyl trimonium chloride led to substantially weaker waves.

### Example 5:

A permanent waving product consisting of two Compositions A and B, filled into a two-chamber packaging the chambers of which were kept separate until application, was prepared by destruction of the separating wall and applied onto human hair rolled onto curlers. The hair was rinsed after about fifteen minutes processing and neutralized for about five minutes with a 2.5 % H₂O₂ neutralizer composition, rinsed again, shampooed and dried.

An expressive, even, intensive permanent wave was obtained.

An identical treatment which had no Dow Corning HMW 2220 and palmitamidopropyl trimonium chloride showed a visibly inferior wave.

**Composition A:**

| | |
|---|---|
| Ammonium hydrogen carbonate | 4.5 (g) |
| Dow Corning HMW 2220 | 1.0 |
| Palmitamidopropyl trimonium chloride | 0.5 |
| PEG-65-Hydrogenated castor oil | 0.8 |
| Isopropyl alcohol | 1.5 |
| Ethoxydiglycol | 2.0 |
| Cocoamidopropyl betaine | 1.0 |
| Perfume | 0.3 |
| Coenzyme Q10 | 0.05 |
| Turbidifying agent | 0.5 |
| Ammonia, 25% | ad pH 8.4 |
| Water | ad 72.0 |

**Composition B:**

| | |
|---|---|
| Ammonium thioglycolate, 70% | 18.0 (g) |
| Thiolactic acid | 2.0 |
| 2-Methyl-1,3-propandiol | 0.5 |
| Ammonia, 25% | ad pH 5.5 |
| Water | ad 28.0 |

After mixing of both Compositions a ready-to-use product with a pH-value of 7.4 was obtained.

### Example 6:

A permanent waving product filled into a two-chamber package was prepared in analogy to Example 4:

**Composition A:**

| | |
|---|---|
| Ammonium hydrogen carbonate | 3.5 (g) |
| Dow Corning HMW 2220 | 0.5 |
| Ethanol | 0.5 |
| 1-Methoxypropanol | 1.0 |
| Cocoamidopropyl betaine | 1.0 |
| PEG-25-glyceryl cocoate | 0.8 |
| Coenzyme Q10 | 0.1 |
| Palmitamidopropyl trimonium chloride | 0.3 |
| Oleic acid | 0.05 |
| Perfume | 0.3 |
| Turbidifying agent | 0.5 |
| Ammonia, 25% | ad pH 8.3 |
| Water | ad 72.0 |

**Composition B:**

| | |
|---|---|
| Ammonium thioglycolate, 70% | 13.0 (g) |
| Thiolactic acid | 0.5 |
| 2-Methyl-1,3-propandiol | 1.5 |
| Ammonia, 25% | ad pH 5.5 |
| Water | ad 28.0 |

A product with a pH-value of 7.4 was obtained by mixing of the Compositions immediately prior to application. After application onto dyed hair as described in Example 3, this mixture resulted in an expressive permanent wave. Hair was soft and smooth and curls had their natural like elasticity and bounce.

### Example 7:

**Alkaline Permanent Waving Gel**

| | |
|---|---|
| Ammonium thioglycolate, 70% | 15.0 (g) |
| Ammonium hydrogen carbonate | 4.5 |
| PEG-40-Hydrogenated castor oil | 0.7 |
| C₁₂-C₁₈-Fatty alcohol mixture | 3.5 |
| Cetrimonium chloride | 1.0 |
| Stearoxyopropyl trimonium chloride | 1.0 |
| Amodimethicone | 0.05 |
| 2-Methyl-1,3-propandiol | 0.5 |
| Dow Corning HMW 2220 | 0.45 |
| Coenzyme Q10 | 0.1 |
| Perfume | 0.3 |
| Ammonia, 25% | ad pH 8.0 |
| Water | ad 100.0 |

**Intermediate treatment composition**

| | |
|---|---|
| Asparagic acid | 0.25% by weight |
| Glutamic acid | 0.50 |
| Alanine DL | 0.25 |
| Magnesium sulfate | 10.00 |
| Dow Corning HMW 2220 | 0.25 |
| Stearoxypropyl trimonium chloride | 0.25 |
| Water | q.s. to 100 |

The above composition had a pH of 4.10.

Hair was waved according to the process as follows: Hair was shampooed and towel dried and put on curlers. Alkaline permanent waving gel of above was applied and processed for 20 min at approximately 40°C and rinsed off with water and towel dried. Subsequently, intermediate treatment composition was applied and processed for 10 min and the hair was neutralized for about five minutes with a 2.5 % H₂O₂ neutralizer composition, rinsed again, shampooed and dried.

### Example 8:

**Straightening Composition**

| | |
|---|---|
| Thioglycolic acid | 8.0 (% by wt.) |
| C₁₆-C₂₂-Fatty alcohol mixture | 3.5 |
| Oleth-50 | 2.5 |
| Laureth-23 | 1.5 |
| Dow Corning HMW 2220 | 0.9 |
| Stearamidopropyl trimonium chloride | 1.0 |
| Ethanol | 5.0 |
| Perfume | 0.6 |
| Monoethanolamine | ad pH 9.3 |
| Water | ad 100.0 |

This composition constitutes an effecting smoothing composition for kinky hair.

## Claims

1. Composition for the permanent shaping of human hair, **characterized in that** it comprises at least one reducing agent, an aqueous emulsion of divinyldimethicone/dimethicone copolymer with an internal phase viscosity of more than 1 x 10⁸ mm²/s measured at 0.01 Hz and at about 25°C and a compound according to general structure
R₁-A-R₂-B
wherein R₁ is a saturated or unsaturated, straight or branched alkyl group with 8 to 24 C atoms, R₂ is a straight or branched alkyl group with 1 to 4 C atoms, A is a group selected from O, 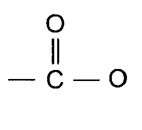 and 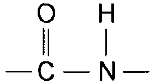 and B is selected from 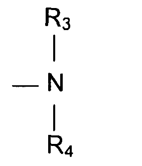 wherein R₃ and R₄ are the same or different is H or an alkyl with 1 to 4 C atoms, hydroxyl alkyl with 1 to 4 C atoms and di hydroxyl alkyl with 2 to 4 C atoms, 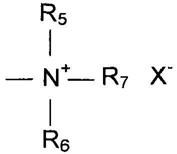 R₅, and R₆ are the same or different, an alkyl with 1 to 4 C atoms, hydroxyl alkyl with 1 to 4 C atoms and di hydroxyl alkyl with 2 to 4 C atoms, R₇ is an alkyl with 1 to 4 C atoms, hydroxyl alkyl with 1 to 4 C atoms or di hydroxyl alkyl with 2 to 4 C atoms and
-R₂-A-R₁
wherein R₁, A and R₂ have the above meaning and X is chloride, bromide, methosulfate.

2. Composition according to claim 1 **characterized in that** the reducing agents are selected from thioglycolic acid, thiolactic acid and their salts, cystein and its hydrochloride salt, acetylcystein.

3. Composition according to claims 1 and 2 **characterised in that** it comprises reducing agent at a concentration of 0.5 to 15% by weight and an aqueous emulsion of divinyldimethicone/dimethicone copolymer at a concentration of 0.1 to 2.5 % by weight and compound according to above given general structure at a concentration of 0.1 to 2.5 % by weight calculated to total composition.

4. Composition according to any of the preceding claims **characterized in that** aqueous emulsion of divinyldimethicone/dimethicone copolymer is a nonionic emulsion and comprises one or more nonionic surfactants.

5. Composition according to any of the preceding claims, **characterized in that** it has a Brookfield viscosity of 1 to 50,000 mPa.s at 20°C.

6. Composition according to any of the preceding claims **characterized in that** it comprises at least one surfactant selected from anionic, nonionic, cationic and amphoteric ones at a concentration of 0.05 to 10% by weight, calculated to total composition, wherein cationic surfactants are selected from surfactants of the general structure 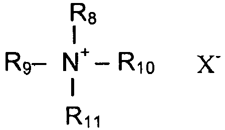 where R₈ is a saturated or unsaturated, branched or non-branched alkyl chain with 8-24 C atoms R₉ is a hydrogen, saturated or unsaturated, branched or non-branched alkyl chain with 1-24 C atoms and R₁₀ and R₁₁ are independent from each other H or lower alkyl chain with 1 to 4 carbon atoms which may be substituted with one or two hydroxyl group, and X is chloride, bromide or methosulfate.

7. Composition according to any of the preceding claims **characterized in that** it comprises at least one organic solvent at a concentration of 0.1 to 10% by weight, calculated to total composition.

8. Composition according to any of the preceding claims **characterized in that** it comprises at least one ubichinone of the formula 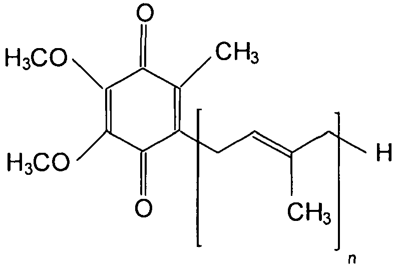 where n is a number between 1 and 10.

9. Composition according to any of the preceding claims **characterized in that** it comprises at least one additional silicone compound, preferably aminated silicone at a concentration of 0.05 to 2.5% by weight, calculated to total composition.

10. Composition according to any of the preceding claims **characterized in that** it comprises at least one fatty alcohol.

11. Composition according to any of the preceding claims **characterized in that** it has a pH in the range of 6.5 to 10.5.

12. Use of composition according to any of the preceding claims for permanent shaping hair.

13. Process for permanent waving hair **characterized in that** hair is washed or shampooed first and wound on curlers and subsequently a composition according to claims 1 to 11 is applied onto hair and at the end of the 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers are removed from hair.

14. Process according to claim 12 **characterized in that** after rinsing off the reducing agent from hair, an intermediate treatment composition, optionally comprising an aqueous emulsion of divinyldimethicone/dimethicone copolymer with an internal phase viscosity of more than 1 x 10⁸ mm²/s measured at 0.01 Hz and at about 25°C and at least one compound according to general structure
R₁-A-R₂-B
wherein R₁ is a saturated or unsaturated, straight or branched alkyl group with 8 to 24 C atoms, R₂ is a straight or branched alkyl group with 1 to 4 C atoms, A is a group selected from O, 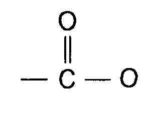 and 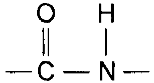 and B is selected from 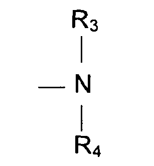 wherein R₃ and R₄ are the same or different is H or an alkyl with 1 to 4 C atoms, hydroxyl alkyl with 1 to 4 C atoms and di hydroxyl alkyl with 2 to 4 C atoms, 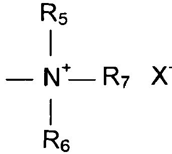 R₅, and R₆ are the same or different, an alkyl with 1 to 4 C atoms, hydroxyl alkyl with 1 to 4 C atoms and di hydroxyl alkyl with 2 to 4 C atoms, R₇ is an alkyl with 1 to 4 C atoms, hydroxyl alkyl with 1 to 4 C atoms or di hydroxyl alkyl with 2 to 4 C atoms and
-R₂-A-R₁
wherein R₁, A and R₂ have the above meaning and X is chloride, bromide, methosulfate is applied onto hair and without rinsing off and after removal of the excess amount with towel, an oxidizing composition is applied and at the end of the processing they are rinsed off from hair.

15. Process for straightening hair **characterized in that** hair is washed and/or shampooed and dried and reducing composition according to claims 1 to 11 is applied onto dry hair and processed for 5 to 60 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

## Patentansprüche

1. Zusammensetzung zum dauerhaften Formen des menschlichen Haares, **dadurch gekennzeichnet, dass** sie mindestens ein Reduktionsmittel, eine wässrige Emulsion von Divinyldimeticon/Dimeticon-Copolymer mit einer Viskosität der inneren Phase von mehr als 1 x 10⁸ mm²/s, gemessen bei 0,01 Hz und bei etwa 25 °C, und eine Verbindung der allgemeinen Struktur
R₁-A-R₂-B
aufweist, wobei R₁ für eine gesättigte oder ungesättigte, geradkettige oder verzweigte Alkylgruppe mit 8 bis 24 C-Atomen steht, R₂ für eine geradkettige oder verzweigte Alkylgruppe mit 1 bis 4 C-Atomen steht, A für eine Gruppe steht, ausgewählt aus O, 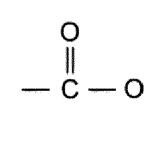 und 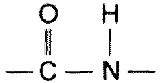 und B ausgewählt ist aus 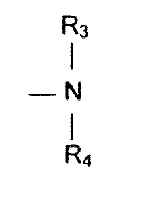 wobei R₃ und R₄ gleich oder verschieden sind und für H oder ein Alkyl mit 1 bis 4 C-Atomen, Hydroxyalkyl mit 1 bis 4 C-Atomen und Dihydroxyalkyl mit 2 bis 4 C-Atomen stehen, 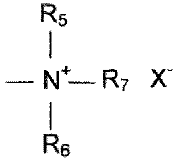 wobei R₅ und R₆ gleich oder verschieden sind und für ein Alkyl mit 1 bis 4 C-Atomen, Hydroxyalkyl mit 1 bis 4 C-Atomen und Dihydroxyalkyl mit 2 bis 4 C-Atomen stehen, R₇ für ein Alkyl mit 1 bis 4 C-Atomen, Hydroxyalkyl mit 1 bis 4 C-Atomen oder Dihydroxyalkyl mit 2 bis 4 C-Atomen steht, und
-R₂-A-R₁
wobei R₁, A und R₂ die obige Bedeutung aufweisen und X für Chlorid, Bromid, Methosulfat steht.

2. Zusammensetzung nach Anspruch 1, **dadurch gekennzeichnet, dass** die Reduktionsmittel aus Thioglykolsäure, Thiomilchsäure und ihren Salzen, Cystein und seinem Hydrochloridsalz, Acetylcystein ausgewählt sind.

3. Zusammensetzung nach Anspruch 1 und 2, **dadurch gekennzeichnet, dass** sie Reduktionsmittel in einer Konzentration von 0,5 Gewichts-% bis 15 Gewichts-% und eine wässrige Emulsion von Divinyldimeticon/Dimeticon-Copolymer in einer Konzentration von 0,1 Gewichts-% bis 2,5 Gewichts-% und eine Verbindung der obigen allgemeinen Struktur in einer Konzentration von 0,1 Gewichts-% bis 2,5 Gewichts-% aufweist, bezogen auf die Gesamtzusammensetzung.

4. Zusammensetzung nach einem der vorhergehenden Ansprüche, **dadurch gekennzeichnet, dass** die wässrige Emulsion von Divinyldimeticon/Dimeticon-Copolymer eine nichtionische Emulsion ist und ein oder mehrere nichtionische Tenside aufweist.

5. Zusammensetzung nach einem der vorhergehenden Ansprüche, **dadurch gekennzeichnet, dass** sie eine Brookfield-Viskosität von 1 mPa·s bis 50.000 mPa s bei 20 °C aufweist.

6. Zusammensetzung nach einem der vorhergehenden Ansprüche, **dadurch gekennzeichnet, dass** sie mindestens ein Tensid, ausgewählt aus anionischen, nichtionischen, kationischen und amphoteren, in einer Konzentration von 0,05 Gewichts-% bis 10 Gewichts-% aufweist, bezogen auf die Gesamtzusammensetzung, wobei kationische Tenside ausgewählt sind aus Tensiden der allgemeinen Struktur 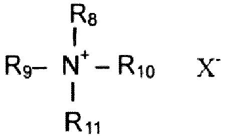 wobei R₈ für eine gesättigte oder ungesättigte, verzweigte oder unverzweigte Alkylkette mit 8 bis 24 C-Atomen steht, R₉ für einen Wasserstoff, eine gesättigte oder ungesättigte, verzweigte oder unverzweigte Alkylkette mit 1 bis 24 C-Atomen steht und R₁₀ und R₁₁ unabhängig voneinander für H oder eine niedere Alkylkette mit 1 bis 4 Kohlenstoffatomen stehen, welche mit einer oder zwei Hydroxylgruppen substituiert sein kann, und X für Chlorid, Bromid oder Methosulfat steht.

7. Zusammensetzung nach einem der vorhergehenden Ansprüche, **dadurch gekennzeichnet, dass** sie mindestens ein organisches Lösungsmittel in einer Konzentration von 0,1 Gewichts-% bis 10 Gewichts-% aufweist, bezogen auf die Gesamtzusammensetzung.

8. Zusammensetzung nach einem der vorhergehenden Ansprüche, **dadurch gekennzeichnet, dass** sie mindestens ein Ubichinon der Formel 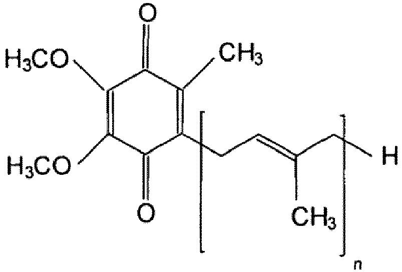 aufweist, wobei n eine Zahl von 1 bis 10 ist.

9. Zusammensetzung nach einem der vorhergehenden Ansprüche, **dadurch gekennzeichnet, dass** sie mindestens eine zusätzliche Silikonverbindung aufweist, vorzugsweise aminiertes Silikon in einer Konzentration von 0,05 Gewichts-% bis 2,5 Gewichts-%, bezogen auf die Gesamtzusammensetzung.

10. Zusammensetzung nach einem der vorhergehenden Ansprüche, **dadurch gekennzeichnet, dass** sie mindestens einen Fettalkohol aufweist.

11. Zusammensetzung nach einem der vorhergehenden Ansprüche, **dadurch gekennzeichnet, dass** sie einen pH-Wert im Bereich von 6,5 bis 10,5 aufweist.

12. Verwendung einer Zusammensetzung nach einem der vorhergehenden Ansprüche zum dauerhaften Formen von Haar.

13. Verfahren zum dauerhaften Wellen von Haar, **dadurch gekennzeichnet, dass** das Haar zuerst gewaschen oder shampooniert wird und auf Lockenwickler gewickelt wird und anschließend eine Zusammensetzung nach Anspruch 1 bis 11 auf das Haar aufgebracht wird und am Ende der 1- bis 45-minütigen, vorzugsweise 1- bis 30-minütigen Einwirkungszeit, die von der Haarstärke abhängt, mit Leitungswasser aus dem Haar ausgespült wird und eine oxidierende Zusammensetzung, die mindestens ein Oxidationsmittel, vorzugsweise Wasserstoffperoxid oder Natriumbromat, in einer Konzentration von 0,5 Gewichts-% bis 10 Gewichts-% aufweist, bezogen auf die gesamte oxidierende Zusammensetzung, auf das Haar aufgebracht wird und 1 bis 20 Minuten lang auf dem Haar gelassen wird und ausgespült wird und die Lockenwickler aus dem Haar entfernt werden.

14. Verfahren nach Anspruch 12, **dadurch gekennzeichnet, dass** nach dem Ausspülen des Reduktionsmittels aus dem Haar eine Zwischenbehandlungszusammensetzung, gegebenenfalls aufweisend eine wässrige Emulsion von Divinyldimeticon/Dimeticon-Copolymer mit einer Viskosität der inneren Phase von mehr als 1 x 10⁸ mm²/s, gemessen bei 0,01 Hz und bei etwa 25 °C, und mindestens eine Verbindung der allgemeinen Struktur
R₁-A-R₂-B
wobei R₁ für eine gesättigte oder ungesättigte, geradkettige oder verzweigte Alkylgruppe mit 8 bis 24 C-Atomen steht, R₂ für eine geradkettige oder verzweigte Alkylgruppe mit 1 bis 4 C-Atomen steht, A für eine Gruppe steht, ausgewählt aus O, 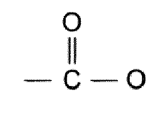 und 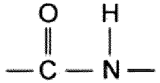 und B ausgewählt ist aus 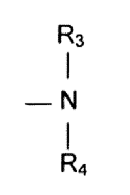 wobei R₃ und R₄ gleich oder verschieden sind und für H oder ein Alkyl mit 1 bis 4 C-Atomen, Hydroxyalkyl mit 1 bis 4 C-Atomen und Dihydroxyalkyl mit 2 bis 4 C-Atomen stehen, 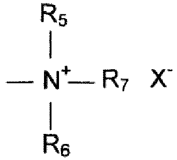 wobei R₅ und R₆ gleich oder verschieden sind und für ein Alkyl mit 1 bis 4 C-Atomen, Hydroxyalkyl mit 1 bis 4 C-Atomen und Dihydroxyalkyl mit 2 bis 4 C-Atomen stehen, R₇ für ein Alkyl mit 1 bis 4 C-Atomen, Hydroxyalkyl mit 1 bis 4 C-Atomen oder Dihydroxyalkyl mit 2 bis 4 C-Atomen steht, und
- R₂-A-R₁
wobei R₁, A und R₂ die obige Bedeutung aufweisen und X für Chlorid, Bromid, Methosulfat steht, auf das Haar aufgebracht wird und ohne Ausspülen und nach dem Entfernen der überschüssigen Menge mit einem Handtuch eine oxidierende Zusammensetzung aufgebracht wird und sie am Ende des Einwirkens aus dem Haar ausgespült werden.

15. Verfahren zum Glätten von Haar, **dadurch gekennzeichnet, dass** das Haar gewaschen und/oder shampooniert und getrocknet wird und eine reduzierende Zusammensetzung nach Anspruch 1 bis 11 auf das trockene Haar aufgebracht wird und man sie für 5 bis 60 Minuten einwirken lässt und sie mit Wasser ausgespült wird und das trockene Haar bei einer Temperatur von 130 °C bis 210 °C mit einem Glätteisen physikalisch geglättet wird und anschließend eine oxidierende Zusammensetzung, die mindestens ein Oxidationsmittel, vorzugsweise Wasserstoffperoxid oder Natriumbromat, in einer Konzentration von 0,5 Gewichts-% bis 10 Gewichts-% aufweist, bezogen auf die gesamte oxidierende Zusammensetzung, auf das Haar aufgebracht wird und 1 bis 20 Minuten lang auf dem Haar gelassen wird und ausgespült wird.

## Revendications

1. Composition pour la mise en forme permanente des cheveux humains, **caractérisée en ce qu'**elle comprend au moins un agent réducteur, une émulsion aqueuse d'un copolymère divinyldiméthicone / diméthicone avec une viscosité de sa phase interne de plus de 1 x 10⁸ mm²/s mesurée à 0,01 Hz et à environ 25 °C et un composé selon la structure générale
R₁-A-R₂-B
dans laquelle R₁ est un groupe alkyle saturé ou non saturé, linéaire ou ramifié, de 8 à 24 atomes de C, R₂ est un groupe alkyle, linéaire ou ramifié, de 1 à 4 atomes de C, A est un groupe choisi parmi un atome d'O, un groupe 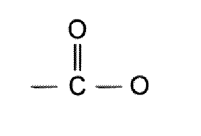 et 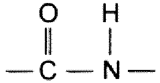 et B est choisi parmi un groupe 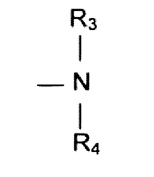 où R₃ et R₄ sont identiques ou différents, sont un atome H ou un groupe alkyle de 1 à 4 atomes de C, un groupe alkyle hydroxylé de 1 à 4 atomes de C et un groupe alkyle di hydroxylé de 2 à 4 C atomes de C, un groupe 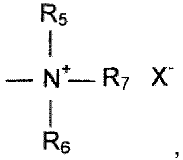 R₅ et R₆ sont identiques ou différents, un groupe alkyle de 1 à 4 atomes de C, un groupe alkyle hydroxylé de 1 à 4 atomes de C et un groupe alkyle di hydroxylé de 2 à 4 atomes de C, R₇ est un groupe alkyle de 1 à 4 atomes de C, un groupe alkyle hydroxylé de 1 à 4 atomes de C ou un groupe alkyle di hydroxylé de 2 à 4 atomes de C, et un groupe
-R₂-A-R₁
où R₁, A et R₂ ont la signification donnée ci-dessus et X est un ion chlorure, bromure, méthosulfate.

2. Composition selon la revendication 1, **caractérisée en ce que** les agents réducteurs sont choisis parmi de l'acide thioglycolique, de l'acide thiolactique et de leurs sels, de la cystéine et de son sel chlorhydrate, de l'acétylcystéine.

3. Composition selon les revendications 1 et 2, **caractérisée en ce qu'**elle comprend un agent réducteur à une concentration en poids de 0,5 à 15 % et une émulsion aqueuse de copolymère divinyldiméthicone / diméthicone à une concentration en poids de 0,1 à 2,5 % et un composé selon la structure générale donnée ci-dessus à une concentration en poids de 0,1 à 2,5 % calculée par rapport à la composition totale.

4. Composition selon l'une quelconque des revendications précédentes, **caractérisée en ce que** l'émulsion aqueuse du copolymère divinyldiméthicone / diméthicone est une émulsion non ionique et comprend un seul ou plusieurs tensio-actifs.

5. Composition selon l'une quelconque des revendications précédentes, **caractérisée en ce qu'**elle a une viscosité Brookfield de 1 à 50.000 mPa à 20 °C.

6. Composition selon l'une quelconque des revendications précédentes, **caractérisée en ce qu'**elle comprend au moins un tensio-actif choisi parmi les tensio-actifs anioniques, cationiques et amphotères à un concentration en poids de 0,05 à 10 %, calculée par rapport à la composition totale, où les tensio-actifs cationiques sont choisis parmi des tensio-actifs de formule générale 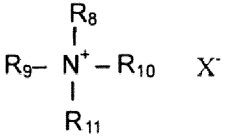 où R₈ est une chaîne alkyle saturée ou non saturée, linéaire ou ramifiée, de 8 à 24 atomes de C, R₉ est un atome d'hydrogène, une chaîne alkyle saturée ou non saturée, linéaire ou ramifiée, de 1 à 24 atomes de C, et R₁₀ and R₁₁ sont indépendamment l'un de l'autre un atome H ou une chaîne alkyle courte avec 1 à 4 atomes de carbone qui peut être substituée par un seul ou deux groupes hydroxyle, et X est un ion chlorure, un ion bromure ou un ion méthosulfate.

7. Composition selon l'une quelconque des revendications précédentes **caractérisée en ce qu'**elle comprend au moins un solvant organique à une concentration en poids de 0,1 à 10 %t, calculée par rapport à la composition totale.

8. Composition selon l'une quelconque des revendications précédentes, **caractérisée en ce qu'**elle comprend au moins une ubiquinone de formule 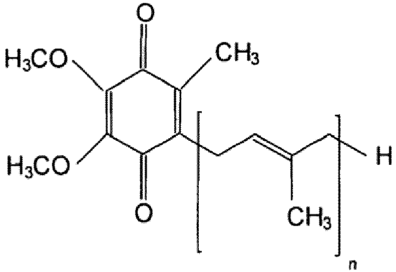 où n est un nombre entre 1 et 10.

9. Composition selon l'une quelconque des revendications précédentes, **caractérisée en ce qu'**elle comprend au moins un composé silicone complémentaire, de préférence un silicone aminé à une concentration en poids de 0,05 à 2,5 %, calculée par rapport à la composition totale.

10. Composition selon l'une quelconque des revendications précédentes **caractérisée en ce qu'**elle comprend au moins un alcool gras.

11. Composition selon l'une quelconque des revendications précédentes, **caractérisée en ce qu'**elle a un pH dans une gamme de 6,5 à 10,5.

12. Utilisation de la composition selon l'une quelconque des revendications précédentes pour une mise en forme permanente des cheveux.

13. Procédé pour une mise en plis permanente des cheveux **caractérisée en ce que** les cheveux sont d'abord lavés et shampouinés et enroulés sur des bigoudis, et ensuite une composition selon les revendications 1 à 11 est appliquée sur les cheveux et après 1 à 45 minutes, de préférence après 1 à 30 minutes de durée de traitement, selon la force des cheveux, la chevelure est rincée à l'eau du robinet et une composition oxydante comprenant au moins un agent oxydant, de préférence du peroxyde d'hydrogène ou du bromate de sodium à une concentration en poids de 0,5 à 10 % calculée par rapport à la totalité de la composition oxydante, est appliquée sur les cheveux et laissée à l'air pendant 1 à 20 minutes et rincée, et les bigoudis sont enlevés des cheveux.

14. Procédé selon la revendication 12, **caractérisé en ce qu'**après le rinçage de l'agent réducteur des cheveux, une composition de traitement intermédiaire, comprenant éventuellement une émulsion aqueuse du copolymère divinyldiméthicone / diméthicone avec une viscosité de sa phase interne de plus de 1 x 10⁸ mm²/s mesurée à 0,01 Hz et à environ 25 °C et au moins un composé selon la formule générale
R₁-A-R₂-B
où R₁ est un groupe alkyle saturé ou non saturé, linéaire ou ramifié, de 8 à 24 atomes de C, R₂ est un groupe alkyle linéaire ou ramifié, de 1 à 4 atomes de C, A est un groupe parmi un atome d'O, un groupe 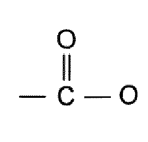 et un groupe 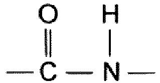 et où B est choisi parmi un groupe 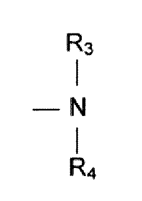 où R₃ et R₄ sont identiques ou différents, sont un atome H ou un groupe alkyle de 1 à 4 atomes de C, un groupe alkyle hydroxylé de 1 à 4 atomes de C et un groupe alkyle di hydroxylé de 2 à 4 C atomes de C, un groupe 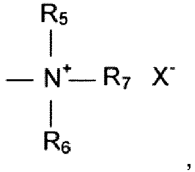 R₅ et R₆ sont identiques ou différents, un groupe alkyle de 1 à 4 atomes de C, un groupe alkyle hydroxylé de 1 à 4 atomes de C et un groupe alkyle di hydroxylé de 2 à 4 atomes de C, R₇ est un groupe alkyle de 1 à 4 atomes de C, un groupe alkyle hydroxylé de 1 à 4 atomes de C ou un groupe alkyle di hydroxylé de 2 à 4 atomes de C, et un groupe
-R₂-A-R₁
où R₁, A et R₂ ont la signification donnée ci-dessus et X est un ion chlorure, un ion bromure, un ion méthosulfate, est appliquée sur les cheveux et sans qu'elle ne soit enlevée par rinçage, mais et après l'enlèvement de la quantité en excès avec une serviette, une composition oxydante est appliquée, et à la fin du traitement elles sont enlevées par rinçage des cheveux.

15. Procédé de lissage des cheveux **caractérisé en ce que** les cheveux sont lavés et/ou shampouinés et séchés et la composition réductrice selon les revendications 1 à 11 est appliqué sur les cheveux secs et agit pendant 5 à 60 minutes et est enlevée par rinçage à l'eau et les cheveux sont séchés, et les cheveux secs sont défrisés physiquement avec un fer chaud à une température de 130 à 210 °C et ensuite une composition oxydante comprenant au moins un agent oxydant, de préférence du peroxyde d'hydrogène ou du bromate de sodium, à une concentration en poids de 0,5 à 10 % calculée par rapport à la totalité de la composition oxydante, est appliquée sur les cheveux et laissée à l'air pendant 1 à 20 minutes et est enlevée par rinçage.
